Europäisches Patentamt

European Patent Office

Office européen des brevets

(19)

(11) Publication number: **0 179 626 B1**

(12) **EUROPEAN PATENT SPECIFICATION**

(45) Date of publication of patent specification: **06.05.92**

(51) Int. Cl.⁵: **A61F 2/30**, A61F 2/36, A61F 2/38

(21) Application number: **85307533.1**

(22) Date of filing: **18.10.85**

(54) Improvements relating to bone implants.

(30) Priority: **24.10.84 GB 8426866**

(43) Date of publication of application:
**30.04.86 Bulletin 86/18**

(45) Publication of the grant of the patent:
**06.05.92 Bulletin 92/19**

(84) Designated Contracting States:
**CH DE FR GB LI**

(56) References cited:
**EP-A- 0 046 926        WO-A-85/03426
DE-A- 2 726 297        DE-A- 2 808 740
FR-A- 2 325 355        FR-A- 2 378 505
FR-A- 2 519 248        FR-A- 2 519 248**

(73) Proprietor: **FINSBURY (INSTRUMENTS) LIMIT-
ED
City Gate House Finsbury Square
London EC2(GB)**

(72) Inventor: **Tuke, Michael Antony
14 Rutland Drive
Morden Surrey SM4 5OH(GB)**
Inventor: **Freeman, Michael Alexander
Reykers
79 Albert Street
London NW1 7LX(GB)**

(74) Representative: **Eyles, Christopher Thomas et
al
W.P. THOMPSON & CO. High Holborn House
52-54 High Holborn
London WC1V 6RY(GB)**

Rank Xerox (UK) Business Services
(-/2.18/2.0)

EP 0 179 626 B1

## Description

This invention relates to prosthetic devices intended for incorporation in the human or animal body and is particularly concerned with such devices in the form of artificial implants, such as implants for replacing hip joints or knee joints.

Several suitable high strength metallic materials for the construction of prosthetic devices such as hip implants have been widely used, e.g. cobalt/chromium alloys. It has been usual for such metallic implants to be held in the bone by means of cements, such as acrylic cements. However, the use of such cements has certain disadvantages and the current trend is to avoid the use of cement by designing implants which are precision fitted so as not to need such cement. Various alternatives allowing one to dispense with cement have been proposed including the provision of roughened surfaces to key to the adjacent bone, ranging from very rough surfaces composed of coarse balls to slightly roughened surfaces. Implants with large holes to encourage bone growth within the holes have also been proposed. However new bone growth is dependent on the age and health of the patient and cannot be expected invariably to take place in a satisfactory manner.

It has been observed that implants with rough surfaces or holes, designed to achieve direct incorporation or so called "in growth", do not always show reliable bone incorporation or consistent strength.

However, in the field of dentistry it has been shown that for insertion of a tooth in the jaw bone via a screw, better integration is obtained if the screw is inserted in an unloaded state several months before the tooth itself is added.

Furthermore the use of cementless implants involves considerable contacting of the metallic material with the bone and this can give rise to toxicity problems. Thus for example it has been found that the constituents of a cobalt/chromium alloy, in itself a very suitable high strength material for an implant, can leach into the body, possibly causing undesirable side effects including cellular modification with possible carcinogenic effects. In addition, the surface treatments of the implants can involve heat treatment which can alter the mechsnical properties of the implant materials and can render them less capable of withstanding the stresses to which they will be subjected in use.

There is clearly a need for the provision of prosthetic devicea which can be readily introduced into the body without the use of cement but which retain their desired mechanical properties and do not have an adverse effect on the body.

DE-A-2726297 describes a two part endoprosthesis including (i) a hip joint part 2 with an articular portion in the form of a ball head H and a stem 6 and (ii) an anchoring part in the form of a sleeve 2. Figure 3 shows how the tapered stem 6 locks together immediately following implantation with the corresponding tapered interior surface of the sleeve 1.

DE-A-2808740 shows in Figure 3 how a hip implant sits in a socket 1 provided with an external screw thread, the space between the inner wall of the socket 1 and the stem 3 of the implant being filled with a suitable binding mass, for example bone cement.

FR-A-2519248 discloses a hip prosthesis comprising a flexible sleeve 3 which the surgeon implants in a prepared intramedullar cavity in the patient's femur and then fills its interior with cement before introducing the stem or tail 19 of the hip implant proper. This cement is then allowed to set in order to provide a seating for the stem or tail 19.

FR-A-2325355 discloses a ring 10, made for example of polyethylene, which has a peripheral flange that sits on the opening to the intramedullar cavity and provides a bearing for a corresponding metallic flange 26 of the hip implant. The metallic stem 14 of the hip implant is then cemented in place.

FR-A-2378505 discloses a partial knee prosthesis in which a tibial plate 3 has a projection 9 which fits in an anchorage well 11 with a flange 11a that bears upon the bone around the cavity formed in the tibia by the surgeon.

There is clearly a need for the provision of prosthetic devices which can readily be introduced into the body without the use of cement but which retain their desired mechanical properties and do not have an adverse effect on the body.

According to this invention we provide a hip implant intended for incorporation in a human or animal body by implantation in a prepared cavity in a femur comprising (i) a first part including the articular portion of the implant and a stem with a substantially linear axis and (ii) a second part in the form of a sleeve intended for positioning with the prepared cavity and dimensioned so as to be capable of snugly receiving the stem of the first part characterised in that the first part comprises: a stem portion, the stem portion having at least in the upper region of one end thereof a substantially circular cross section; a ball head; and a wedge-shaped portion extending medially from the stem portion at or near the opposite end to said one end, the wedge-shaped portion being surmounted by the ball head, and that the sleeve is open at both ends and includes a slot which is intended to accommodate the wedge-shaped portion and which extends part way only along the length of the sleeve from one end thereof.

The outer surface of the sleeve may conveniently be screw threaded to promote its insertion in the bone and subsequent integration therewith. However, the outer surface may be provided with other types of surface to promote integration or, yet again, the sleeve may be a simple press-fit within an accurately reamed cavity.

The sleeve is preferably of pure titanium which is tolerated well by the body. However other suitably inert materials may be used, such as ceramics. The screw thread or other integration means, such as ridges or grooves, may extend over the whole or part of the sleeve. A thread preferably has a rounded edge so as not to damage the bone in which it is to be inserted. The sleeve may be in one or more parts. The thread may have a size, shape and pitch dependent on the particular bone in which it is to be used. Single, multiform and discontinuous threads may be used. By such variations the optimum load bearing area can be achieved for a particular implant in a particular type of bone.

The first part may be of any suitable material with the required strength properties for manufacture of prosthetic devices. Suitable materials are cobalt/chromium alloys, stainless steel, titanium or tantalum. Plastic components can also be fitted in this way. The first and second parts should of course be of mutually compatible materials which are electrochemically inert under the saline conditions to be found in the body.

The implant may be in the form of a hip implant, suitably of the type described in our copending European application No. 85302469.3 (Publication No. 0158534). Thus the first part preferably comprises a stem portion, with a substantially linear axis, having, at least in the region of one end thereof, a substantially circular cross-section and a wedge-shaped portion extending medially from the stem portion at or near the opposite end to the said one end, the wedge-shaped portion being surmounted by a ball.

The invention will now be described by way of example with reference to the accompanying drawings wherein:-

Figure 1 is a side view of a first part of a hip implant in accordance with the invention;

Figure 2 is a side view of part of the hip implant showing also a second part thereof; and

Figure 3 is a perspective view of the second part of the hip implant of Figure 2.

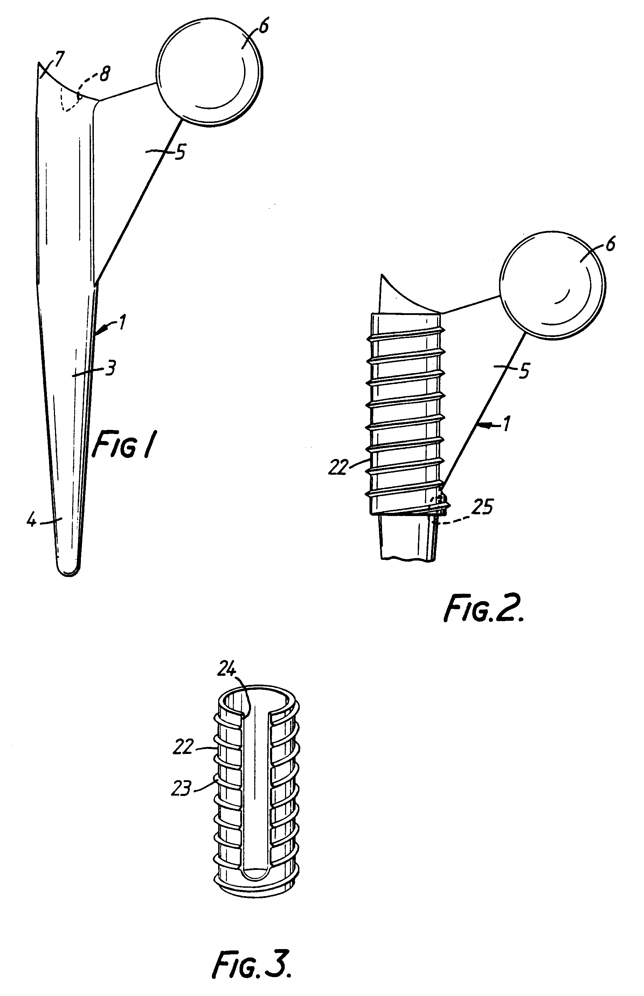

The hip implant shown in Figures 1 to 3 is of the type described in our copending European application No. 85302469.3 (Publication No. 0158534). However it is in two parts, a first part 1 (shown in Figure 1) including the articular portion, and a second part provided by a sleeve 22 shown in Figures 2 and 3. Part 1 is of an acceptable material for introduction to the human body such as a cobalt/chromium alloy or titanium alloy. It may be all of one material or may have a ball head portion of a different material such as a ceramic. The part 1 comprises a stem 3 which has a substantially linear axis and a substantially circular cross-section which tapers towards its lower end 4. Spaced from the tapered portion 4, a flattened neck portion 5 extends medially from the stem 3 and is surmounted by a ball head 6. The stem 3 extends beyond the neck portion 5 to form a projecting end 7 for lateral support and provided with an aperture 8, which, in use, can receive a hook or like means for removing the implant from the bone if this becomes necessary.

The second part 22 of the implant is in the form of an open-ended sleeve provided with a screw thread 23 and a slot 24 to accommodate neck portion 5 of part 1. It can be made of an inert material, preferably pure titanium. In use, sleeve 22 is inserted in the bone and part 1 is initially spaced therefrom as shown at 25 but gradually sinks into the sleeve by a distance of, for example 1 to 2 mm, over a period of perhaps 6 months, by which time the sleeve 22, as it is now integrated with the surrounding bone, is itself in a position to bear load better and prevent further sinkage of the first part of the implant. The load is thus applied to the bone at a higher portion of the hip.

The sleeve 22 of Figures 2 and 3 may be inserted into the femur by any suitable technique. Thus the sleeve 22 may be screwed into the bone so that the outer surface is in close contact with surrounding cancellous bone. Bone-sleeve contact may be enhanced by packing with ground bone paste to facilitate grafting.

If necessary the part 1 can be removed from the sleeve 22 and, as the sleeve 22 is screw threaded, it can be unscrewed from the bone. The thread is shaped and dimensioned and of a pitch chosen to suit a particular type of bone so as to ensure that optimum load is eventually borne by the implant and adjacent bone. The size, pitch and form of the screw thread can be used to vary the area of bone implant contact and load carriage.

It will be seen that the design of a first part and outer sleeve of the present invention is such that, while positional control is maintained via the sleeve, the sleeve is at least initially essentially not loaded in compression (the main load carriage of the joint). Other areas of the implant are thus initally responsible for principal load carriage. The relatively unloaded sleeve will then be better able to integrate with bone during this period. With later sinkage of the first part of the implant within the sleeve, the first part will then engage on the sleeve and obtain support via the sleeve which will then bear some of the load. However by this time good bone integration with the sleeve will have occurred making the sleeve more satisfactorily load bearing and minimising further sinkage.

## Claims

1. A hip implant intended for incorporation in a human or animal body by implantation in a prepared cavity in a femur comprising (i) a first part (1) including the articular portion of the implant and a stem with a substantially linear axis and (ii) a second part in the form of a sleeve (22) intended for positioning with the prepared cavity and dimensioned so as to be capable of snugly receiving the stem of the first part characterised in that the first part (1) comprises: a stem portion (3), the stem portion (3) having, at least in the upper region of one end thereof a substantially circular cross section; a ball head (6); and a wedge-shaped portion (5) extending medially from the stem portion at or near the opposite end to said one end, the wedge-shaped portion being surmounted by the ball head (6) and that the sleeve (22) is open at both ends and includes a slot (24) which is intended to accommodate the wedge-shaped portion (5) and which extends part way only over the length of the sleeve (22) from one end thereof.

2. A hip implant according to claim 1, characterised in that the sleeve (22) is made from titanium.

3. A hip implant according to claim 1 or claim 2, characterised in that the sleeve (22) is screw threaded.

4. A hip implant according to claim 3, characterised in that the screw thread has a rounded edge.

5. A hip implant according to any one of claims 1 to 4, characterised in that the stem portion (3) has its lower end conically tapered.

## Revendications

1. Prothèse de la hanche destinée à être insérée dans un corps humain ou animal par implantation dans une cavité fémorale préparée comprenant (i) une première partie (1) comportant la partie articulée de la prothèse et une tige d'axe essentiellement linéaire et (ii) une deuxième partie accusant la forme d'une gaine (22) destinée à être placée dans la cavité préparée et dont les dimensions sont telles qu'elle puisse bien recevoir la tige de la première partie caractérisée en ce que la première partie (1) comporte: une tige (3), cette tige (3) présentant, au moins à la section supérieure en une extrémité de celle-ci, une coupe transversale essentiellement circulaire; une tête fémorale (6); ainsi qu'une partie cunéiforme (5) d'extension médiane à partir de la tige au côté opposé ou près du côté opposé à ladite extrémité, la partie cunéiforme étant surmontée de la tête fémorale (6) et en ce que la gaine (22) est ouverte en ses deux extrémités et comporte une fente (24) destinée à recevoir la partie cunéiforme (5) et qui ne couvre que partiellement la longueur de la gaine (22) à partir d'une extrémité de celle-ci.

2. Prothèse de la hanche selon la revendication 1, caractérisée en ce que la gaine (22) est en titane.

3. Prothèse de la hanche selon la revendication 1 ou la revendication 2, caractérisée en ce que la gaine (22) présente un filetage de vis.

4. Prothèse de la hanche selon la revendication 3, caractérisée en ce que le filetage de vis présente un rebord arrondi.

5. Prothèse de la hanche selon l'une quelconque des revendications 1 à 4, caractérisée en ce que la tige (3) présente une extrémité inférieure à biseautage conique.

## Patentansprüche

1. Hüftimplantat zum Einbau in den Körper eines Menschen oder Tieres durch Implantation in eine vorbereitete Aushöhlung in einem Femur, bestehend aus (i) einem ersten Teil (1), der den Gelenkabschnitt des Implantats und einen Stiel mit einer im wesentlichen linearen Achse umfaßt, und (ii) einem zweiten Teil in Form einer Hülse (22), die zum Einsetzen mit der vorbereiteten Aushöhlung bestimmt und so dimensioniert ist, daß der Stiel des ersten Teils satt darin sitzen kann, dadurch gekennzeichnet, daß der erste Teil (1) umfaßt: einen Stielabschnitt (3), wobei der Stielabschnitt (3), zumindest im oberen Bereich eines seiner Enden, einen im wesentlichen kreisförmigen Querschnitt aufweist; einen kugelförmigen Kopf (6); und einen keilförmigen Abschnitt (5), der sich medial von dem Stielabschnitt an dem oder nahe bei dem dem genannten Ende entgegengesetzten Ende erstreckt, wobei der keilförmige Abschnitt von dem kugelförmigen Kopf (6) überragt wird, und daß die Hülse (22) an beiden Enden offen ist und einen Schlitz (24) aufweist, der für die Aufnahme des keilförmigen Abschnitts (5) vorgesehen ist und der sich nur über einen Teil der Länge der Hülse (22), ausgehend von einem ihrer Enden, erstreckt.

2. Hüftimplantat nach Anspruch 1, dadurch gekennzeichnet, daß die Hülse (22) aus Titan besteht.

3. Hüftimplantat nach Anspruch 1 oder Anspruch 2, dadurch gekennzeichnet, daß die Hülse (22) ein Schraubengewinde aufweist.

4. Hüftimplantat nach Anspruch 3, dadurch gekennzeichnet, daß das Schraubengewinde eine gerundete Kante aufweist.

5. Hüftimplantat nach einem der Ansprüche 1 bis 4, dadurch gekennzeichnet, daß sich das untere Ende des Stielabschnitts (3) konisch verjüngt.

Fig 1

FIG.2.

FIG.3.